# EUROPÄISCHE PATENTSCHRIFT

(11) **EP 1 986 696 B1**
(45) Veröffentlichungstag und Bekanntmachung des Hinweises auf die Patenterteilung: **11.01.2012**
(21) Anmeldenummer: 07712266.1
(22) Anmeldetag: 21.02.2007
(51) Int. Cl.: A61K 48/00, A61K 47/48, A61K 47/34, C07H 21/00, C08G 65/00, C12N 15/00

(54) **EIN VERFAHREN ZUR HERSTELLUNG EINER TRANSFORMIERTEN ZELLE**
METHOD FOR PRODUCTION OF A TRANSFORMED CELL
PROCÉDÉ DE PRODUCTION D'UNE CELLULE TRANSFORMÉE

(30) Priorität: 23.02.2006 DE 102006008701
(43) Veröffentlichungstag der Anmeldung: 05.11.2008
(73) Patentinhaber: Qiagen GmbH, 40724 Hilden (DE)
(72) Erfinder: ERBACHER, Christoph, 42781 Haan (DE); KRÜGER, Ute, 42653 Solingen (DE)
(74) Vertreter: Hüttermann, Aloys
(86) Internationale Anmeldenummer: PCT/EP2007/051661
(87) Internationale Veröffentlichungsnummer: WO 2007/096382

(56) Entgegenhaltungen:
- EP-A- 1 552 818
- WO-A-2004/078822
- WO-A1-00/27795
- DATABASE CAPLUS CHEMICAL ABSTRACTS SERVICE, COLUMBUS, OHIO, US; 1999, QU R. ET AL.: "Adsorption property and mechanism of polymeric aza-crown ether resin for metallic ions" XP007904004 & QU RONGJUN ET AL: "Adsorption property and mechanism of polymeric aza-crown ether resin for metallic ions" GAOFENZI CAILIAO KEXUE YU GONGCHENG, SICHUAN DAXUE, GAOFENZI YANJIUSUO,, CN, Bd. 15, Nr. 2, Mai 1999 (1999-05), Seiten 151-154, XP008088326 ISSN: 1000-7555
- NGUYEN H K ET AL: "EVALUATION OF POLYETHER-POLYETHYLENEIMINE GRAFT COPOLYMERS AS GENE TRANSFER AGENTS" GENE THERAPY, MACMILLAN PRESS LTD., BASINGSTOKE, GB, Bd. 7, Januar 2000 (2000-01), Seiten 126-138, XP002947334 ISSN: 0969-7128
- BANERJEE P. ET AL.: "Linear Polyethyleneimine Grafted to a Hyperbranched Poly(ethylene glycol)-like Core: A Copolymer for Gene Delivery" BIOCONJUGATE CHEMISTRY, Bd. 17, 22. Dezember 2005 (2005-12-22), Seiten 125-131, XP007904002
- DATABASE PUBMED [Online] 01 Februar 2006 BOOJAR MM, GOODARZI F.: 'Cytotoxicity and the levels of oxidative stress parameters in WI38 cells following 2 macrocyclic crown ethers treatment' Database accession no. 16153622

## Beschreibung

Die vorliegende Erfindung betrifft ein in vitro Verfahren zum Einschleusen eines Biomoleküls in eine Zelle, die in vitro Verwendung eines bestimmten Polymers zum Einschleusen eines Biomoleküls in eine Zelle, einen Kit zum in vitro Einschleusen eines Biomoleküls in eine Zelle, die in vitro Verwendung dieses Kits zum Einschleusen eines Biomoleküls in eine Zelle, einen Komplex aus einem Biomolekül und einem Polymer, eine therapeutische Zusammensetzung.

Aus dem Stand der Technik sind zahlreiche unterschiedliche Methoden bekannt, um Fremd-Nukleinsäuren, insbesondere Fremd-DNA, in eine Zelle einzubringen (Transformation oder Transfektion). Zur *Transformation in vivo* werden im Wesentlichen zwei Methoden angewendet, Virus-vermittelter Gentransfer und die Transformation mittels kationischer Liposomen oder kationischer Polymere, wie etwa Polyethyleniminen, Poly-L-Lysin oder Poly-L-Arginin. Als virale Vektoren werden u.a. Adenoviren und Retroviren benutzt, die in der Lage sind, nichtvirale DNA in mitotisch aktive Zellen zu transfizieren. Die kationischen Liposomen interagieren mit dem negativ geladenen Phosphat-Rückgrat der DNA.

Zur Transformation *in vitro* eignet sich die Calcium-Phosphat-Präzipitation, bei der sich in einem Gemisch aus Calciumchlorid und Natriumphosphat die zu übertragende DNA an das ausfallende Calciumphosphat bindet. Die ausgefallenen Kristalle werden der Zellkultur zugegeben und von den Zellen durch Endocytose aufgenommen. Neben diesem chemischen Transformationsverfahren sind auch zahlreiche physikalische *in vitro*-Transformationsverfahren bekannt, wie etwa die Mikroinjektion, bei der die DNA mit einer Injektionsapperatur direkt in den Zellkern bzw. in die Zelle injiziert wird, die Elektroporation, bei der die Zellmembran durch Elektroschocks für DNA permeabel gemacht wird sowie das sogenannte *"Particle Gun*"-Verfahren, bei dem die Fremd-DNA mit Hilfe von winzigen Metallkügelchen, auf denen die DNA fixiert wurde, in die Zelle geschossen wird.

Der Nachteil der vorstehend beschriebenen *in vitro*- Transformationsverfahren liegt insbesondere darin, dass die Verfahren häufig sehr zellspezifisch sind. Bei der Calcium-Phosphat-Präzipitation ist der Transformationsvorgang zudem stark abhängig von der Wahl der richtigen Salzkonzentrationen und erfordert von Zellart zu Zellart experimentelles Geschick und Erfahrung. Von größerer Bedeutung ist jedoch der Nachteil der aus dem Stand der Technik beschriebenen Transformationsverfahren, dass diejenigen Transformationsreagenzien, die eine ausreichende Transformationseffizienz aufweisen, häufig stark cytotoxisch sind, während diejenigen Reagenzien, die keine oder allenfalls geringe cytotoxische Eigenschaften aufweisen, häufig nur durch eine unbefriedigende Transformationseffizienz gekennzeichnet sind.

Um diese Nachteile zu überwinden, werden alternativ kationische Polymere zur *in vitro*-Transformation bzw. Transfektion von Zellen eingesetzt. So ist beispielsweise bekannt, dass die Komplexierung von DNA mit Polyethylenimin (PEI) erfolgreich eingesetzt werden kann, um Gene in die Zelle zu transportieren (Boussif et al., 1995; Boussif et al., 1996; Abdallah et al., 1996). Der Gentransfer erfolgt dabei dadurch, dass die Komplexe ungerichtet an Zellen gebunden und aufgenommen werden. Neben Polyethyleniminen werden auch Homopolymere basischer Aminosäuren, wie etwa Poly-L-Lysin, dendritische kationische Polymere, wie etwa Polyamidoamine (PAMAMs), Polyamide, Polyallylamine, kationische Methacrylate, Chitosan oder Poly(D,L-Lactid-co-Glycolid) (PLGAs) als kationische Polymere zur Transfektion von Zellen eingesetzt. Eine Übersicht über kationische Polymere, welche zur Transfektion von Zellen eingesetzt werden können, bieten beispielsweise Holtorf und Mikos in "Cationic and non-condensing polymer-based gene delivery", Pharmaceutical Perspectives of Nucleic Acid-Based Therapeutics, 2002 (183), Seiten 367-387.

Qu R. et Al. Gaofenzi Cailiao Kexue Yu Gongcheng, Sichuan Daxue, Gaofenzi Yanjiusuo, 15(2), 1999, 151-154 offenbart ein Aza-Kronenether-Harz, welches durch Quervernetzung von Ethylenglycoldiglycidyl-Ether mit Polyaminen hergestellt wird. Diese Harze werden zu Abwasserreinigung benutzt.

EP1552818 offenbart Polymere zum Einsatz bei der verzögerten Freisetzung von Stoffen. WO2004/078822 offenbart Sulfonsäure-enthaltende Polymere, die im Rückgrat durch die nukleophile Öffnung von Epoxiden durch Amine herstellbar sind, und für elektrochemische Prozesse eigesetzt werden.

Nguyen H. K. et Al., Gene Therapy, 2000, 7, 126-138, und Banerjee P. et Al., Bioconjugate Chemistry 2006, 17, 125-131, offenbaren den Einsatz von Polyimin-Polyether-Polymeren bei der Gentherapie.

Der Nachteil dieser aus dem Stand der Technik bekannten kationischen Polymere liegt entweder darin, dass sie eine nur geringe Transfektionseffizienz aufweisen oder aber, dass sie zwar durch eine zufrieden stellende Transfektionseffizienz gekennzeichnet sind, jedoch häufig stark cytotoxisch wirken. Zudem ist bei einigen der aus dem Stand der Technik bekannten Polymere, insbesondere bei den dendritischen Polymeren, der Syntheseaufwand unverhältnismäßig hoch. So liegt der Zeitaufwand für eine 42 kDa PAMAM-Dendrimer-Synthese bei vielen Wochen.

Der vorliegenden Erfindung lag die Aufgabe zugrunde, die sich aus dem Stand der Technik ergebenden Nachteile bei der Transformation von Zellen, insbesondere bei der *in vitro*-Transformation, zu überwinden.

Ferner lag der vorliegenden Erfindung die Aufgabe zugrunde, ein Verfahren zur Herstellung einer transformierten Zelle anzugeben, wobei das Verfahren durch eine hohe Transformationseffizienz gekennzeichnet ist. Zudem sollte das Verfahren möglichst ohne den Einsatz cytotoxischer Hilfsstoffe durchgeführt werden können.

Auch lag der vorliegenden Erfindung die Aufgabe zugrunde, ein Verfahren zur Herstellung einer transformierten Zelle anzugeben, bei dem die zur Transformation eingesetzten Hilfsstoffe unter möglichst geringem Syntheseaufwand an die Besonderheiten der zu transformierten Zellpopulationen und den jeweiligen Transformationsbedingungen angepasst werden können. Auch sollten die eingesetzten Hilfsstoffe möglichst leicht und kostengünstig herstellbar sein.

Einen Beitrag zur Lösung der eingangs genannten Aufgaben leistet ein Verfahren zum Einschleusen eines Biomoleküls in eine Zelle in vitro, beinhaltend die Verfahrensschritte:
i) Herstellen eines Komplexes aus einem Biomolekül, vorzugsweise einer Nukleinsäure, und einem Polymer, welches erhältlich ist durch Reaktion eines Amin-Monomers, aufweisend mindestens zwei Amin-Gruppen, mit einem Epoxid-Monomer, aufweisend mindestens zwei Epoxid-Gruppen, sowie
ii) Einschleusen des Biomoleküls in eine Zelle durch das in Kontakt bringen einer Zelle mit dem Komplex.

Völlig überraschend wurde festgestellt, dass sich mittels eines Polymers, welches durch Polyadditionsreaktion eines Diamins mit einem Diepoxid hergestellt werden kann, Biomoleküle mit hoher Effizienz bei nur geringer cytotoxischer Belastung in Zellen einschleusen lassen.

Im Verfahrensschritt i) des erfindungsgemäßen Verfahrens wird zunächst ein Komplex aus einem Biomolekül und einem Polymer, welches erhältlich ist durch die Reaktion eines Amin-Monomers, aufweisend mindestens zwei Amin-Gruppen, mit einem Epoxid-Monomer, aufweisend mindestens zwei Epoxid-Gruppen, bereitgestellt.

Vorzugsweise handelt es sich bei dem Biomolekül, welches im Verfahrensschritt i) zur Herstellung des Komplexes eingesetzt wird, um eine Nukleinsäure, so dass sich das erfindungsgemäße Verfahren zur Transformation einer Zelle eignet. Der Begriff "Transformation", wie er hierin verwendet wird, beschreibt ganz allgemein den Vorgang des Einführens von Biomolekülen, insbesondere von Nukleinsäuren, vorzugsweise von Desoxyribonukleinsäure (DNA), in beliebige Zellen. Er umfasst insbesondere auch den Sonderfall der Transfektion, bei der Fremd-DNA in eine Zellkulturzelle eingeführt wird. Der Begriff "Transformation" umfasst sowohl das nur zeitweilige Einbringen einer DNA, beispielsweise eines Gens, in die Zelle (transiente Transformation), als auch den dauerhaften Einbau der DNA in das Genom der Zelle (stabile Transformation).

Die Nukleinsäure, die im Verfahrensschritt i) zur Herstellung des Komplexes eingesetzt wird und für die synonym auch der Begriff Polynukleotid verwendet werden kann, kann eine Ribonukleinsäure (RNA), Deoxyribonukleinsäure (DNA), oder auch gemischte Form sein, wobei eine Desoxyribonukleinsäure besonders bevorzugt ist. Im allgemeinen Fall kann es sich um jeden Typ von Polynukleotid handeln, der ein N-Glykosid oder C-Glykosid einer Purin- oder Pyrimidinbase oder einer modifizierten Purin- oder Pyrimidinbase darstellt, also auch solche Basen enthalten kann, die in der Natur nicht vorkommen. Außer der Base selbst kann auch der Zuckerrest modifiziert sein. Eingeschlossen sind Nukleinsäuren mit modifizierter Verknüpfung der Untereinheiten, etwa Phosphorothioat- oder -amidat-Verknüpfungen. Die Nukleinsäure kann, einzel-, doppel- oder mehrsträngig, linear oder zirkulär sein. Sie kann einem in einer Zelle vorkommenden Molekül entsprechen, wie etwa genomische DNA oder Boten-RNA (mRNA), oder *in vitro* erzeugt werden wie Komplementär-DNA (cDNA), Gegenstrang-RNA (aRNA), oder synthetische Nukleinsäuren. Die Nukleinsäure kann aus wenigen Untereinheiten, mindestens zwei Untereinheiten, bevorzugt acht oder mehr Untereinheiten bestehen, wie etwa Oligonukleotide, mehreren hundert Untereinheiten bis hin zu mehreren tausend Untereinheiten; wie etwa bestimmte Expressionsvektoren. Bevorzugt enthält die Nukleinsäure die kodierende Information für ein Polypeptid in funktionellem Zusammenhang mit regulatorischen Sequenzen, die die Expression des Polypeptids in der Zelle erlauben, in die die Nukleinsäure eingebracht wird. So ist die Nukleinsäure in einer bevorzugten Ausführungsform ein Expressionsvektor. In einer anderen Ausführungsform ist sie eine pDNA (Plasmid-DNA), eine siRNA, eine siRNA-Duplizes, eine siRNA-hetero-Duplizes oder eine miR-NA, wobei unter dem Begriff "siRNA" Ribonukleinsäuren mit einer Länge von etwa 22 Nukleotiden verstanden werden, die durch Spaltung einer doppelsträngigen RNA (dsRNA) durch das Enzym "Dicer" entstehen und in den Enzymkomplex "RISC"-(RNA-induced silencing complex) eingebaut werden. Auch synthetische siRNAs können als Biomoleküle im Verfahrensschritt i) eingesetzt werden.

Neben Nukleinsäuren kommen als Biomoleküle auch Oligopeptide, Proteine oder Lipide in Betracht.

Das im Verfahrensschritt i) eingesetzte Polymer ist erhältlich durch Reaktion eines Amin-Monomers, aufweisend mindestens zwei Amin-Gruppen, mit einem Epoxid-Monomer, aufweisend mindestens zwei Epoxid-Gruppen.

Gemäß einer bevorzugten Ausführungsform des erfindungsgemäßen Verfahrens weist das Amin-Monomer mindestens zwei funktionelle Gruppen der Struktur I 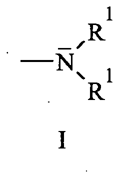 auf, worin die Reste R¹ innerhalb der funktionellen Gruppe gleich oder verschieden sein können und einen C₁- bis C₂₀-Kohlenwasserstoff-Rest, besonders bevorzugt einen C₁- bis C₁₀-Kohlenwasserstoff Rest und am meisten bevorzugt einen C₁- bis C₅-Kohlenwasserstoff-Rest, insbesondere einen Methyl-Rest, einen Ethyl-Rest, einen n-Propyl-Rest oder einen iso-Propyl-Rest, einen hydroxyfunktionellen C₁- bis C₂₀-Kohlenwasserstoff-Rest, besonders bevorzugt einen hydroxyfunktionellen C₁- bis C₁₀-Kohlenwasserstoff-Rest und am meisten bevorzugt einen hydroxyfunktionellen C₁- bis C₅-Kohlenwasserstoff-Rest oder aber ein Wasserstoffatom darstellen. Weiterhin ist es erfindungsgemäß bevorzugt, dass mindestens einer der Reste R¹ innerhalb einer funktionellen Gruppe der Struktur I ein Wasserstoffatom ist. Der Begriff "Kohlenwasserstoff-Rest" umfasst dabei sowohl Reste, die ausschließlich aus Kohlenstoffatomen und Wasserstoffatomen bestehen als auch Reste, die neben Kohlenstoffatomen und Wasserstoffatomen auch Heteroatome, insbesondere Atome aus der Gruppe der Halogene ( = halogenierte Kohlenwasserstoffe) umfassen.

Bei der funktionellen Amin-Gruppe I kann es sich demnach um eine primäre Amin-Gruppe, eine sekundäre Amin-Gruppe oder aber um eine tertiäre Amin-Gruppe handeln, wobei Amin-Monomere mit mindestens zwei primären Amin-Gruppen (in diesem Fall sind beide Reste R¹ ein Wasserstoffatom) am meisten bevorzugt sind. Besonders bevorzugte Reste R¹ sind die Ethyl-Gruppe, die Methyl-Gruppe, die -CH₂CH₂OH-Grupp sowie das Wasserstoffatom.

Weiterhin ist es erfindungsgemäß bevorzugt, dass das Amin-Monomer die Struktur II 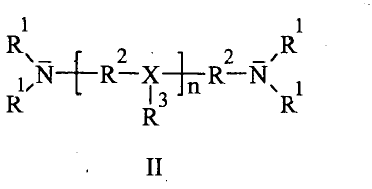 aufweist, in der
- X: sofern vorhanden, ein Sauerstoffatom oder ein Stickstoffatom, besonders bevorzugt ein Sauerstoffatom oder ein Stickstoffatom ist, wobei im Falle eines Sauerstoffatoms die R³-Gruppe am Atom X nicht vorhanden ist und wobei die Atome X innerhalb eines Amin-Monomers auch verschieden sein können,
- -R¹-: innerhalb der Struktur II gleich oder verschieden sein kann und einen C₁-bis C₂₀-Kohlenwasserstoff-Rest, besonders bevorzugt einen C₁- bis C₁₀-Kohlenwasserstoff-Rest und am meisten bevorzugt einen C₁- bis C₅-Kohlenwasserstoff-Rest, insbesondere einen Methyl-Rest, einen Ethyl-Rest, einen n-Propyl-Rest oder einen iso-Propyl-Rest, einen hydroxyfunktionellen C₁- bis C₂₀-Kohlenwasserstoff-Rest, besonders bevorzugt einen hydroxy-funktionellen C₁- bis C₁₀-Kohlenwasserstoff-Rest und am meisten bevorzugt einen hydroxyfunktionellen C₁- bis C₅-Kohlenwasserstoff-Rest oder aber ein Wasserstoffatom darstellt,
- R²: innerhalb der Struktur II gleich oder verschieden sein kann und eine C₁-bis C₂₀-Alkylen-Gruppe, besonders bevorzugt eine C₁-bis C₁₀-Alkylen-Gruppe und am meisten bevorzugt eine C₁-bis C₆-Alkylen-Gruppe oder eine C₁-bis C₂₀₀-Oxyaklen-Gruppe, besonders bevorzugt-eine C₁-bis C₅₀-Oxyalkylen-Gruppe und am meisten bevorzugt eine C₂-bis C₁₀-Oxyalkylen-Gruppe darstellt, wobei als Rest R² die Reste -CH₂CH₂-, -CH₂CH₂CH₂- und-CHCH₃CH₂-, besonders bevorzugt und -CH₂CH₂- am meisten bevorzugt ist,
- n: wenn R² eine Alkylen-Gruppe ist, eine ganze Zahl aus dem Bereich von 0 bis 6, besonders bevorzugt von 1 bis 5, darüber hinaus bevorzugt von 1 bis 3 und am meisten bevorzugt von 1 bis 3 ist, und, wenn R² eine Oxyalkylen-Gruppe ist, eine ganze Zahl aus dem Bereich von 0 bis 100, besonders be- vorzugt von 1 bis 50, darüber hinaus bevorzugt von 2 bis 25 und am meisten bevorzugt von 2 bis 10 ist, und
- R³: innerhalb der Struktur II gleich oder verschieden sein kann und einen C₁-bis C₂₀-Kohlenwasserstoff-Rest, besonders bevorzugt einen C₁- bis C₁₀-Kohlenwasserstoff-Rest und am meisten bevorzugt, einen C₁- bis C₅-Kohlenwasserstoff-Rest, insbesondere einen Methyl-Rest, einen Ethyl-Rest, einen n-Propyl-Rest oder einen iso-Propyl-Rest, einen hydroxyfunktionellen C₁- bis C₂₀-Kohlenwasserstoff-Rest, besonders bevorzugt einen hydroxy-funktionellen C₁- bis C₁₀-Kohlenwasserstoff-Rest und am meisten bevorzugt einen hydroxyfunktionellen C₁- bis C₅-Kohlenwasserstoff-Rest, ein Wasserstoffatom oder einen Rest der Struktur III 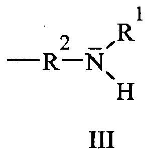 darstellt, wobei als Rest R³ der Rest -CH₂-CH₂-NR¹H, insbesondere mit R¹ = H und R² = -CH₂CH₂-, am meisten bevorzugt ist.

Erfindungsgemäß besonders bevorzugte Amin-Monomere, welche die vorstehend dargestellte Struktur II aufweisen, sind ausgewählt aus der Gruppe bestehend aus den folgenden Monomeren a bis n:

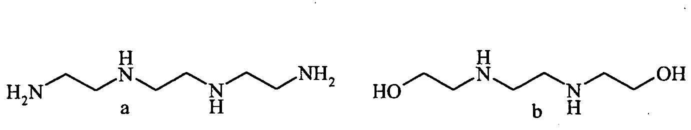

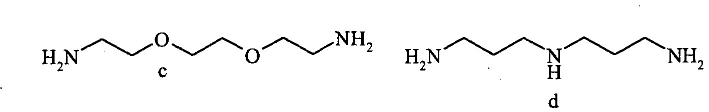

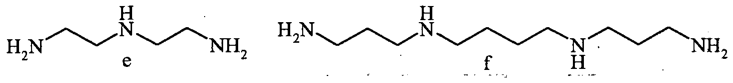

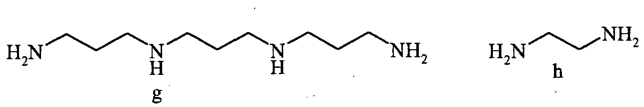

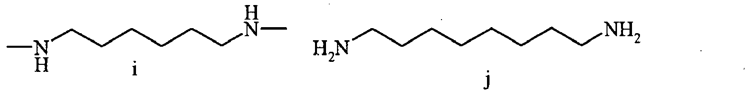

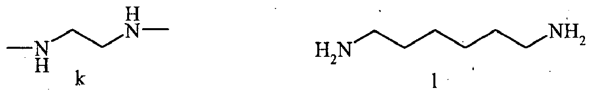

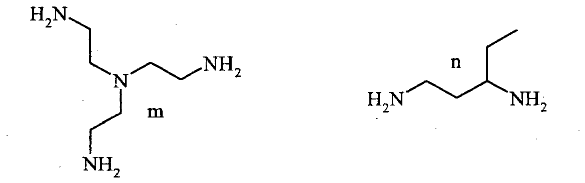

Neben den vorstehend beschriebenen Amin-Monomeren mit der Struktur II können gemäß einer anderen Ausführungsform des erfindungsgemäßen Verfahrens zur Herstellung der Polymere auch Amin-Monomere eingesetzt werden, welche ein gegebenenfalls Heteroatom-substituiertes, aromatisches Ringsystem, besonders bevorzugt ein gegebenenfalls Heteroatom-substituiertes aromatisches C₆-oder C₁₀-Ringsystem umfassen, an das mindestens zwei funktionelle Gruppen der Struktur I 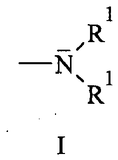 gebunden sind, worin R¹ wie vorstehend definiert ist.

Unter einem "aromatischen Ringsystem" wird vorzugsweise jedes ringförmige System aus sp²-hybridisierten Kohlenstoffatomen verstanden, welches der Hückel-Regel genügt, wobei an die Kohlenstoffatome gegebenenfalls auch ein oder mehrere Halogenatome, wie Fluor oder Chlor (= halogenierte Aromaten), eine oder mehrere Alkyl-Gruppen, insbesondere Methyl-Gruppen, oder aber eine oder mehrere Hydroxyl-Gruppen gebunden sein können.

Bei dem zur Herstellung des Polymers eingesetzten Epoxid-Monomer handelt es sich vorzugsweise um ein Epoxid-Monomer, welches mindestens zwei funktionelle Gruppen der Struktur IV 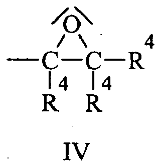 aufweist, worin die Reste R⁴ innerhalb der Struktur IV gleich oder verschieden sein können und einen C₁- bis C₂₀-Kohlenwasserstoff-Rest, besonders bevorzugt einen C₁- bis C₁₀-Kohlenwasserstoff-Rest und am meisten bevorzugt einen C₂- bis C₅-Kohlenwasserstoff-Rest, insbesondere einen Methyl-Rest, einen Ethyl-Rest, einen n-Propyl-Rest oder einen iso-Propyl-Rest, einen hydroxyfunktionellen C₁-bis C₂₀-Kohlenwasserstoff-Rest, besonders bevorzugt einen hydroxyfunktionellen C₁- bis C₁₀-Kohlenwasserstoff-Rest und am meisten bevorzugt einen hydroxyfunktionellen C₂- bis C₅-Kohlenwasserstoff-Rest oder ein Wasserstoffatom darstellen, wobei ein Wasserstoffatom als Rest R⁴ am meisten bevorzugt ist.

Erfindungsgemäß besonders bevorzugte Epoxid-Monomere weisen die Struktur V 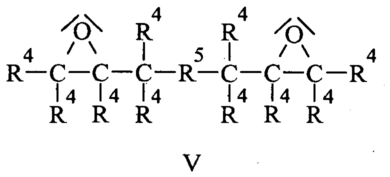 auf, in der
- R⁴: wie vorstehend definiert ist, und
- R⁵: sofern vorhanden, eine C₁- bis C₂₀-Alkylen-Gruppe, besonders bevorzugt eine C₁- bis C₁₀-Alkylen-Gruppe und am meisten bevorzugt eine C₁-bis C₆-Alkylen-Gruppe, darstellt, wobei -CH₂CH₂-, -CH₂CH₂CH₂- oder -CHCH₃CH₂- als Alkylen-Gruppe besonders bevorzugt und -CH₂CH₂-am meisten bevorzugt ist, oder eine Gruppe der Struktur VI 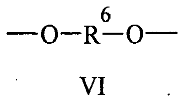 ist, in der R⁶
eine C₁- bis C₂₀-Alkylen-Gruppe, besonders bevorzugt eine C₁- bis C₁₀-Alkylen-Gruppe und am meisten bevorzugt eine C₁-bis C₆-Alkylen-Gruppe darstellt, wobei
-CH₂CH₂-,
-CH₂CH₂CH₂-,
-CHCH₃CH₂-,
[-CH₂CH₂-]ₙ mit n = 1 bis 10,
[-CH₂CH₂CH₂-]ₙ mit n = 1 bis 10,
[-CHCH₃CH₂-]ₙ mit n = 1 bis 10,
   oder ein Polyalkylen umfassend Ethylen- und PropylenEinheiten als Alkylen-Gruppe besonders bevorzugt ist,
oder eine C₁- bis C₂₀₀-Oxyalkylen-Gruppe, besonders bevorzugt eine C₂- bis C₁₀₀-Oxylkylen-Gruppe und am meisten bevorzugt eine C₂-bis C₅-Oxyalkylen-Gruppe darstellt, wobei
[-CH₂CH₂-O-]ₙCH₂- mit n = 2 bis 100,
[-CH₂CH₂CH₂-O-]ₙCH₂CH₂CH₂- mit n = 2 bis 100,
[-CHCH₃CH₂-O-]ₙCHCH₃CH₂- mit n = 2 bis 100,
oder ein Polyoxyalkylen umfassend Oxyethylen- und Oxypropylen-Einheiten als Oxyalkylen-Gruppe besonders bevorzugt ist.

Erfindungsgemäß besonders bevorzugte Epoxid-Monomere, welche die vorstehend dargestellte Struktur V aufweisen, sind ausgewählt aus der Gruppe bestehend aus den folgenden Monomeren A bis F:

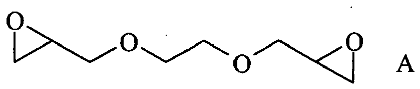

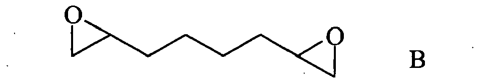

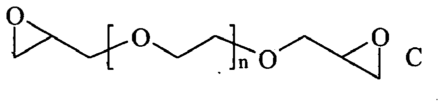

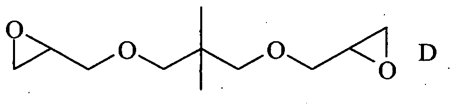

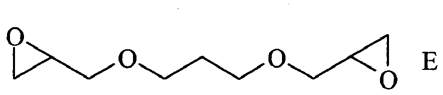

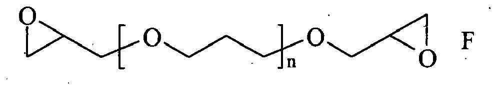

Erfindungsgemäß bevorzugte, im Verfahrensschritt i) zur Herstellung des Komplexes eingesetzte Polymere sind insbesondere solche, welche durch Reaktion folgender Monomer-Komponenten erhältlich sind: aA, aB, aC, aD, aE, aF, bA, bB, bC, bD, bE, bF, cA, cB, cC, cD, cE, cF, dA, dB, dC, dD, dE, dF, eA, eB, eC, eD, eE, eF, fA, fB, fC, fD, fE, fF, gA, gB, gC, gD, gE, gF, hA, hB, hC, hD, hE, hF, iA, iB, iC, iD, iE, iF, jA, jB, jC, jD, jE, jF, kA, kB, kC, kD, kE, kF, lA, lB, lC, lD, lE, lF, mA, mB, mC, mD, mE, mF, nA, nB, nC, nD, nE, nF, oA, oB, oC, oD, oE, oF, qA, qB, qC, qD, qE, qF, rA, rB, rC, rD, rE, rF, sA, sB, sC, sD, sE, sF, tA, tB, tC, tD, tE, tF, uA, uB, uC, uD, uE, uF, vA, vB, vC, vD, vE, vF, wA, wB, wC, wD, wE und wF.

Bei der Reaktion zwischen dem Amin-Monomer und dem Epoxid-Monomer handelt es sich um eine Additionsreaktion, bei der ein Polymer gebildet wird, welches, sofern ein Amin-Monomer mit primären oder sekundären Amin-Gruppen vorlag, mindestens zwei Struktureinheiten der Struktur VII 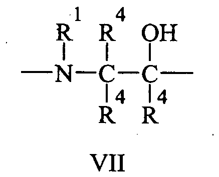 aufweist, und in der R¹ und R⁴ wie vorstehend definiert sind.

Wurde hingegen ein Amin-Monomer eingesetzt, welches tertiäre Amin-Gruppen umfasst hat, so wird ein Polymer gebildet, welches mindestens zwei Struktureinheiten der Struktur VIII 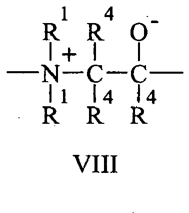 aufweist, in der R¹ und R⁴ ebenfalls wie vorstehend beschrieben definiert sind.

Die Synthese des Polymers durch Polyadditionsreaktion aus dem Amin-Monomer und dem Epoxid-Monomer kann in der dem Fachmann für diese Polymerisationsreaktion bekannten Art und Weise erfolgen. Dabei wird der Fachmann die Reaktionsbedingungen, insbesondere die Art des Lösungsmittels, die Konzentration der Edukte (Amin-Monomer und Epoxid-Monomer), die Reaktionstemperatur und die Reaktionsdauer je nach den gewünschten Eigenschaften des Zielproduktes, insbesondere je nach gewünschtem Molekulargewicht, durch einfache Vorversuche ermitteln.

Üblicherweise erfolgt die Umsetzung derart, dass sowohl das Amin-Monomer als auch das Epoxid-Monomer zunächst in einem geeigneten Lösungsmittel gelöst oder dispergiert, vorzugsweise gelöst werden, wobei vorzugsweise solche Lösungsmittel eingesetzt werden, in denen sich sowohl das Amin-Monomer als auch das Epoxid-Monomer zumindest teilweise lösen. Beispiele für geeignete Lösungsmittel sind Wasser, Alkohole, wie Methanol, Ethanol, 1-Propanol, 2-Propanol, n-Butanol, tert.-Butanol oder iso-Butanol, Ether, Ketone, Kohlenwasserstoffe, Halogenkohlenwasserstoffe, Alkoxyalkanole, vorzugsweise Alkoxypropanol, Alkylpyrrolidone, beispielsweise N-Methylpyrrolidon, Dialkylsulfoxide, beispielsweise Dimethylsulfoxid, oder Mischungen aus mindestens zwei dieser Lösungsmittel.

Die Konzentration der einzelnen Monomere in dem Lösungsmittel liegt zu Beginn der Adduktbildung üblicherweise in einem Bereich von 1 bis 10.000 mMol/l, besonders bevorzugt in einem Bereich von 10 bis 5.000 mMol/l, darüber hinaus noch mehr bevorzugt 50 bis 1.000 mMol/l und am meisten bevorzugt 100 bis 500 mMol/l.

Weiterhin kann es, sofern ein Polymer aus Amin-Monomeren mit der gleichen Struktur und aus Epoxid-Monomeren mit der gleichen Struktur hergestellt werden soll, vorteilhaft sein, dass das Amin-Monomer und das Epoxid-Monomer in einem Molverhältnis Amin-Monomer : Epoxid-Monomer von 2 : 1 bis 1 : 2, besonders bevorzugt 1,5 : 1 bis 1 : 1,5 und am meisten bevorzugt in einem Molverhältnis von etwa 1 : 1 eingesetzt werden.

Durchgeführt wird die Additionsreaktion üblicherweise bei einer Temperatur in einem Bereich von 10 bis 200°C, besonders bevorzugt 20 bis 150°C und am meisten bevorzugt bei 50 bis 100°C, wobei die Dauer der Reaktion und auch die für die Reaktion erforderliche Reaktionstemperatur im Wesentlichen von der Reaktivität der Monomere abhängen. Üblicherweise dauert die Additionsreaktion etwa 0,5 bis 10 Stunden, besonders bevorzugt etwa 1 bis 5 Stunden. Üblicherweise wird die Reaktion bei einem Einsatz eines Amin-Monomers mit primären Amin-Gruppen solange durchgerührt, bis keine primären Amin-Gruppen mehr nachzuweisen sind, während bei einem Einsatz eines Amin-Monomers mit sekundären oder tertiären Amin-Gruppen die Reaktion solange durchgerührt wird, bis keine sekundären oder tertiären Amin-Gruppen mehr nachweisbar sind.

Gemäß einer besonderen Ausführungsform des erfindungsgemäßen Verfahrens ist das im Verfahrensschritt i) zur Herstellung des Komplexes eingesetzte Polymer durch Reaktion zweier strukturell verschiedener Amin-Monomere mit einem Epoxid-Monomer erhältlich. So können beispielsweise bei dieser besonderen Ausfiihrungsform des erfindungsgemäßen Verfahrens ein hydrophiles und ein hydrophobes Amin-Monomer eingesetzt werden.

Bei der Verwendung zweier strukturell verschiedener Amin-Monomere sind bei der Herstellung des Polymers zwei Vorgehensweisen denkbar. Zum einen können beide Amin-Monomere zusammen mit dem Epoxid-Monomer in einem geeigneten Lösungsmittel gelöst oder suspendiert werden, wobei es in diesem Fall zu einer statistischen Verteilung der Monomere innerhalb des Polymers kommt, sofern beide Amin-Monomere in etwa die gleiche Reaktivität gegenüber dem Epoxid-Monomer aufweisen. Zum anderen kann jedoch auch zunächst das eine Amin-Monomer mit dem Epoxid-Monomer im Unterschuss umgesetzt werden, wobei das Molverhältnis Amin-Monomer: Epoxid-Monomer höchstens 0,5 : 1, besonders bevorzugt höchstens 0,25 : 1 und am meisten bevorzugt höchstens 0,1 : 1 beträgt. Auf diese Weise werden Polymere erhalten, an deren Kettenende eine reaktive Epoxid-Gruppe vorliegt. Nach der Umsetzung des ersten Amin-Monomers mit dem Epoxid-Monomer wird das so erhaltene Polymere von den übrigen Komponenten der Reaktionsmischung, insbesondere den noch vorhandenen Epoxid-Monomeren, abgetrennt, wobei diese Abtrennung durch dem Fachmann bekannte Trennverfahren, wie beispielsweise Extraktion, Destillation, Kristallisation oder Dialyse, besonders bevorzugt durch Dialyse, erfolgen kann. Anschließend wird das Polymer, sofern es nach der Abtrennung von den noch nicht abreagierten Monomeren als Reinstoff vorliegt, wieder in einem geeigneten Lösungsmittel gelöst und mit dem zweiten Amin-Monomer in Kontakt gebracht, so dass sich nunmehr das zweite Amin-Monomer an die endständigen Epoxid-Gruppen des Polymers anlagert.

Auf die gleiche Art und Weise ist es selbstverständlich auch möglich, ein Polymer aus zwei strukturell verschiedenen Epoxid-Monomeren und einem Amin-Monomer herzustellen. Denkbar ist weiterhin der Einsatz von mindestens zwei, mindestens drei, mindestens vier oder mindestens fünf strukturell verschiedenen Amin-Monomeren und/oder der Einsatz von mindestens zwei, mindestens drei, mindestens vier oder mindestens fünf strukturell verschiedenen Epoxid-Monomeren.

Nach Abschluss der Polyadditionsreaktion liegt ein in dem eingesetzten Lösungsmittel gelöstes oder dispergiertes Polymer neben noch nicht abreagierten Amin- und/oder Epoxid-Monomeren vor. Vor dem Einsatz des Polymers zur Herstellung des Komplexes im Verfahrensschritt i) des erfindungsgemäßen Verfahrens ist es daher bevorzugt, das Reaktionsgemisch aufzuarbeiten, insbesondere das Polymer von den übrigen Komponenten der Reaktionsmischung abzutrennen. Als Trennverfahren kommen auch hier insbesondere die Extraktion, die Destillation, die Kristallisation oder die Dialyse in Betracht, wobei die Dialyse besonders bevorzugt ist, denn durch die Dialyse wird das Polymer unmittelbar in einem Lösungsmittel gelöst oder dispergiert erhalten. Zudem erlaubt die Dialyse, je nach Wahl der Dialysemembran, die gezielte Isolierung eines Polymers mit einer bestimmten Größe. Neben dem Abtrennen des Polymers von den übrigen Komponenten des Reaktionsgemisches ist es auch bevorzugt, dass Polymer bzw. im Falle der Dialyse, die Zusammensetzung aus dem Polymer und einem Lösungsmittel zu sterilisieren, wobei auch hier alle dem Fachmann bekannten Sterilisationsverfahren angewendet werden können, wie etwa die Sterilfiltration, die γ-Bestrahlung, die Bestrahlung mit UV-Licht oder das Autoklavieren, wobei die Sterilfiltration am meisten bevorzugt ist.

Gemäß einer besonders bevorzugten erfolgt das Abtrennen des Monomers von den übrigen Komponenten der Reaktionsmischung und das anschließende Sterilisieren des Polymers, in dem zunächst die Reaktionsmischung mit Wasser in einem Volumenverhältnis Reaktionsmischung : Wasser in einem Bereich von 5 : 1 bis 1 : 5, besonders bevorzugt 2 : 1 bis 1 : 2 und am meisten bevorzugt von etwa 1 : 1 verdünnt und die so erhaltene, verdünnte Reaktionsmischung anschließend mittels einer Dialysemembran mit einem *"molecular weight cut off"* von höchstens 10 kDa, besonders bevorzugt höchstens 5 kDa und am meisten bevorzugt höchstens 1 kDa vorzugsweise gegen ein wässriges Lösungsmittel, beispielsweise gegen vollentsalztes Wasser, gegen eine physiologische Kochsalzlösung oder gegen ein Nährmedium, dialysiert wird. Aus der auf diese Weise erhaltenen, wässrigen Polymerlösung wird, sofern gegen Wasser dialysiert wurde, anschließend das Wasser vorzugsweise mittels Gefriertrocknung angetrennt, wobei in diesem Fall das reine Polymer erhalten werden kann. Dieses Polymer wird anschließend vorzugsweise in einem wässrigen Medium, beispielsweise in vollentsalztem Wasser, physiologischen Salzlösungen wie PBS oder in einem Nährmedium resuspendiert und anschließend sterilfiltriert. Die auf diese Weise erhalte, sterile wässrige Polymerlösung beinhaltet das Polymer vorzugsweise in einer Konzentration in einem Bereich von 0,1 bis 500 mg/ml, besonders bevorzugt 0,5 bis 100 mg/ml und am meisten bevorzugt von 1 bis 10 mg/ml. Denkbar ist auch, die direkt im Anschluss an die Dialyse erhaltene, vorzugsweise wässrige Polymerlösung unmittelbar zu sterilisieren. UK: für die in vitro Anwendung muss das Reagenz steril sein.

Das mittels Massenspektrometrie bestimmte Molekulargewicht des im Verfahrensschritt i) eingesetzten-Polymers liegt vorzugsweise in einem Bereich von 0,1 bis 500 kDa, besonders bevorzugt 1 bis 100 kDa und am meisten bevorzugt 10 bis 50 kDa. Insbesondere durch die Variation der Reaktionszeit, der Konzentration der Monomere, des Lösungsmittels, der Reaktionstemperatur, des Molverhältnisses aus Amin-Monomer zu Epoxid-Monomer und der Ausschlussgröße der Dialysemembran lässt sich das mittlere Molekulargewicht des Polymers in einfacher Weise regulieren und somit auch die Transformationseffizienz für die jeweils beabsichtigte Transformation optimieren.

Weiterhin kann es gemäß einer besonderen Ausführungsform des erfindungsgemäßen Verfahrens auch vorteilhaft sein, das Polymer vor oder auch nach der Abtrennung von den übrigen Komponenten der Reaktionsmischung chemisch zu modifizieren, wobei die Modifizierung vorzugsweise an den im Polymer vorhandenen Amino-Gruppen oder Hydroxyl-Gruppen erfolgt. Erfindungsgemäß bevorzugte Modifizierungen sind solche Modifizierungen, welche die Aufnahme des Polymers in eine Zelle begünstigen. Erfindungsgemäß bevorzugte Modifizierungen umfassen das Anbinden einer Biotin-Gruppe, wie diese beispielsweise in der DE-A-100 16 881 beschrieben ist. Der Offenbarungsgehalt dieser Druckschrift hinsichtlich der Möglichkeiten, Nukleinsäuren durch das Anbinden von Effektorgruppen an kationische Polymere gezielt in Zellen einzuführen, wird hiermit als Referenz eingeführt und bildet einen Teil der Offenbarung der vorliegenden Erfindung. Neben Biotin-Gruppen können die Amin-Gruppen oder die Hydroxyl-Gruppen auch mit anderen Effektorgruppen, wie etwa mit Avidin oder Streptavidin, mit Liganden, die an bestimmte Zellrezeptoren binden, wie etwa mit Transferrin oder mit Kohlenhydraten, insbesondere mit Galaktosiden, oder aber mit Peptiden mit Protein-Transductor-Domainen modifiziert sein. Weiterhin können im Polymer durch die Behandlung mit Glycidol endständige Diolgruppen gebildet oder durch die Behandlung mit Bromessigsäure oder deren Salzen negativ geladene Gruppen im Polymer erzeugt werden. Weiterhin können die im Polymer vorhandenen Amino-Gruppen durch Behandlung mit Alkylierungsmitteln, wie etwa Dimethylsulfat, Methylbromid, Methyltosylat oder Methylmesylat zumindest teilweise quarternisiert werden. Denkbar ist auch, die Amino-Gruppen zumindest teilweise in Arginin-Gruppen zu transformieren.

Der im Verfahrensschritt i) hergestellte Komplex aus dem Biomolekül und dem Polymer wird durch das in Kontakt bringen des Biomoleküls mit dem Polymer erhalten, wobei der Aufbau des Komplexes über elektrostatische Wechselwirkungen erfolgt. Gegebenenfalls kann das Biomolekül, sofern es sich um eine Nukleinsäure handelt, vor dem in Kontakt bringen mit dem Polymer zunächst noch durch Inkubation mit geeigneten Enhancem, insbesondere mit Polypeptiden, wie etwa Protaminsulfat aus Lachssperma und dergleichen, kondensiert werden. Denkbar ist auch das in Kontakt bringen des Biomoleküls mit Peptiden um die Aufnahme in die Zelle zu erleichtern. Denkbar ist weiterhin, dass das Polymer in Kombination mit einem anderen Transformationsmittel, beispielsweise einem liposomalen oder nicht-liposomalen Lipid oder aber in Mischung mit chemisch unterschiedlichen Polymeren, zum Einschleusen eines Biomoleküls, insbesondere einer Nukleinsäure, in eine Zelle eingesetzt wird.

Vorzugsweise wird das Biomolekül mit dem Polymer in einem Gewichtsverhältnis Biomolekül : Polymer in einem Bereich von 10 : 1 bis 1 : 50, besonders bevorzugt in einem Bereich von 1 : 1 bis 1 : 30 und am meisten bevorzugt in einem Bereich von 1 : 2 bis 1 : 20 in Kontakt gebracht. Der Fachmann kann durch Routineexperimente die optimierten Mischungsverhältnisse ermitteln. Bevorzugt wird das Verhältnis Polymer : Biomolekül, wenn es sich bei dem Biomolekül um eine Nukleinsäure handelt, im Falle eines positiven Ladungsüberschusses so eingestellt, dass das Zeta-Potential etwa +20 bis +50 mV beträgt.

Das in Kontakt bringen des Biomoleküls mit dem Polymer erfolgt vorzugsweise in einer wässrigen Lösung, beispielsweise einer physiologischen Kochsalzlösung, einem Puffer oder einem Nähnnedium, wobei das in Kontakt bringen besonders bevorzugt bei einem pH-Wert in einem Bereich von 5 bis 8, besonders bevorzugt von 5,5 bis 7,5 und bei einer Temperatur in einem Bereich von 4 bis 40°C, besonders bevorzugt von 10 bis 37°C und am meisten bevorzugt von 15 bis 25°C erfolgt. Zweckmäßig kann dieses in Kontakt bringen durch Vereinigung wässriger Lösungen der Komponenten ausgeführt werden, wobei eine oder beide dieser Lösungen auch gepuffert sein und ggf. weitere Zusätze enthalten können. Besonders bevorzugt erfolgt das in Kontakt bringen unter Bedingungen, unter denen der mit dem Biomolekül gebildete Komplex bei physiologischer Salzkonzentration nicht dissoziiert, was beispielsweise, wenn es sich bei dem Biomolekül um eine Nukleinsäure handelt, durch den Ethidiumbromidverdrängungsassay (*Ethidiumbromide Displacement Assay*) auf einfache Weise bestimmt werden kann (Plank et al. 1999).

Im Verfahrensschritt ii) des erfindungsgemäßen Verfahrens wird der vorstehend beschriebene Komplex aus dem Biomolekül und dem Polymer mit einer Zelle unter Erhalt einer Zelle, in die ein Biomolekül eingeschleust wurde, in Kontakt gebracht, wobei dieses in Kontakt bringen *in vivo* und *in vitro* erfolgen kann, eine *in vitro*-Behandlung der Zellen jedoch besonders bevorzugt ist. Dabei ist es grundsätzlich möglich, dass der Komplex aus dem Biomolekül und dem Polymer in Gegenwart der Zellen gebildet wird (und somit die Verfahrensschritte i) und ii) zeitgleich durchgeführt werden). Denkbar und bevorzugt ist aber, dass die Komplexbildung in Abwesenheit der zu behandelnden Zellen erfolgt, so dass der Verfahrensschritt ii) im Anschluss an den Verfahrensschritt i) durchgeführt wird.

Bei der *in vitro*-Behandlung können Zellen oder Gewebe mit dem Komplex in einem geeigneten Milieu inkubiert werden, wobei als Zellen sowohl Prokaryonten als auch Eukaryonten in Betracht kommen. Sollen Kulturzellen behandelt werden, kann der Komplex bzw. die sterile wässrige Lösung beinhaltend den Komplex zum Kulturmedium gegeben werden. Geeignet ist das erfindungsgemäße Verfahren sowohl für Zellen, die in Zellkultur in Suspension wachsen (Suspensionszellen) als auch für Zellen, die an Substratoberflächen adhärent wachsen (adhärente Zellen).

Zur Behandlung *in vivo* kann der Komplex bzw. die sterile wässrige Lösung beinhaltend den Komplex in einen tierischen oder pflanzlichen Organismus einschließlich des Menschen appliziert werden. Die Applikation kann dabei systemisch oder topisch erfolgen, bei tierischen Organismen insbesondere parenteral, z. B. durch intravenöse, intraperitoneale, intramuskuläre, intra- oder subkutane Injektion, intravenöse Infusion, pulmonare, buccale, nasale, dermale oder transdermale Applikation. Auch andere Applikationswege, z. B. orale/enterale Applikation sind unter geeigneten Bedingungen und mit entsprechenden Formulierungen möglich. Der Komplex bzw. die sterile wässrige Lösung beinhaltend den Komplex kann auch lokal direkt in ein Zielgewebe, etwa ein Organ oder ein Tumor, appliziert werden. Zur Applikation *in vivo* sollte der Komplex in pharmazeutisch verträglicher Form vorliegen. Er kann dafür mit einem pharmazeutisch akzeptablen Träger kombiniert werden. Die Formulierung richtet sich dabei nach dem Applikationsmodus. Zur Injektion oder Infusion kann der Komplex beispielsweise in isotonischer Kochsalzlösung oder in einem isotonischen Puffer wie PBS oder Ringers Lösung vorliegen, zur pulmonaren Verabreichung zum Beispiel in Form eines pharmazeutisch verträglichen Aerosols. Auch feste Formulierungen können je nach Verwendungszweck gewählt werden, beispielsweise gefriergetrocknete Präparate, die vor Verabreichung mit Wasser rekonstituiert werden können. Dem Fachmann sind geeignete pharmazeutische Träger und Formulierungen bekannt, zum Beispiel aus Gennaro (Hrsg.), Remington: The Science and Practice of Pharmacy, 19. Ausgabe, 1995, Mack Publishing Co., Easton, PA, USA. Auf diese Weise kann der Komplex bei Verwendung einer entsprechenden Nukleinsäure als Biomolekül als Arzneimittel in der Gentherapie eingesetzt werden.

Die Dosierung wählt der Fachmann in Abhängigkeit von der Art der Erkrankung, dem Zustand des Patienten, dem therapeutischen Ansatz sowie weiteren Faktoren. Im Allgemeinen wird, wenn es sich bei dem Biomolekül um eine Nukleinsäure handelt, bei humaner Anwendung die Menge der verabreichten DNA im Bereich von 0.1 µg/kg bis 100 µg/kg Körpergewicht liegen, vorzugsweise 2.5 µg/kg bis 10 µg/kg Körpergewicht.

Einen Beitrag zur Lösung der eingangs genannten Aufgaben leistet weiterhin eine Zelle, in die ein Biomolekül durch das vorstehend beschriebene Verfahren eingeschleust worden ist.

Auch die Verwendung des vorstehend beschriebenen Polymers, welches erhältlich ist durch die Reaktion des Amin-Monomers, aufweisend mindestens zwei Amino-Gruppen, mit dem Epoxid-Monomer, aufweisend mindestens zwei Epoxid-Gruppen, zum Einschleusen eines Biomoleküls in eine Zelle, vorzugsweise zur Transformation einer Zelle, leistet einen Beitrag zur Lösung der eingangs genannten Aufgaben.

Einen weiteren Beitrag zur Lösung der eingangs genannten Aufgaben leistet auch ein Kit zum Einschleusen eines Biomoleküls in eine Zelle, vorzugsweise zur Transformation einer Zelle, umfassend
(β1) das vorstehend beschriebene Polymer, welches erhältlich ist durch die Reaktion des Amin-Monomers, aufweisend mindestens zwei Amin-Gruppen, mit dem Epoxid-Monomer, aufweisend mindestens zwei Epoxid-Gruppen, sowie
(β2) weitere Kit-Komponenten, ausgewählt aus der Gruppe umfassend: Protaminsulfat aus Lachssperma PBS physiologische Kochsalzlösung und/oder liposomale oder nicht-liposomale Lipide.

In Abhängigkeit vom jeweils zu transfizierenden Zelltyp kann eine Kombination aus Polymer mit Lipiden oder Liposomen oder aber eine Mischung diverser Polymere sinnvoll sein. So könnte auch die Verwendung einer Mischung auf Basis dieser Patentanmeldung generierter, aber chemisch unterschiedlicher Polymere verwendet werden.

Einen weiteren Beitrag zur Lösung der eingangs genannten Aufgaben leistet ein Komplex aus einem Biomolekül, vorzugsweise einer Nukleinsäure, und dem vorstehend beschriebenen Polymer, welches erhältlich ist durch Reaktion eines Amin-Monomers, aufweisend mindestens zwei Amin-Gruppen, mit einem Epoxid-Monomer, aufweisend mindestens zwei Epoxid-Gruppen. Dieser Komplex ist beispielsweise erhältlich durch das in Kontakt bringen des Biomoleküls und des Polymers in einem geeigneten wässrigen System, wie es eingangs im Zusammenhang mit dem erfindungsgemäßen Verfahren beschrieben worden ist.

Einen Beitrag zur Lösung der eingangs genannten Aufgaben leistet auch eine therapeutische Zusammensetzung, beinhaltend den vorstehend beschriebenen Komplex aus einem Biomolekül, vorzugsweise einer Nukleinsäure, und dem Polymer, welches erhältlich ist durch die Reaktion des Amin-Monomers, aufweisend mindestens zwei Amin-Gruppen, mit dem Epoxid-Monomer, aufweisend mindestens zwei Epoxid-Gruppen. Neben diesem Komplex kann die therapeutische Zusammensetzung insbesondere auch einen pharmazeutisch akzeptablen Träger, wie etwa eine physiologische Kochsalzlösung, beinhalten. Ebenso leistet auch die Verwendung des vorstehend beschriebenen Komplexes zur Herstellung einer solchen pharmazeutischen Zusammensetzung einen Beitrag zur Lösung der eingangs genannten Aufgaben.

Die vorliegende Erfindung wird nun anhand Beispiele und Figuren näher erläutert.

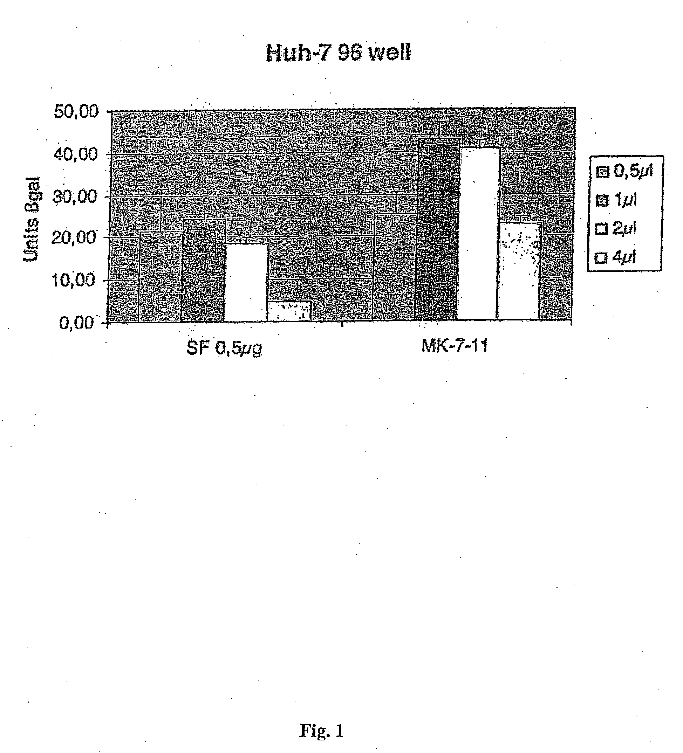

Es zeigt die Figur 1 die Transfektionseffizienz bei der Transfektion von Huh-7-Zellen mit einem pCMVβ-Plasmid gemäß dem erfindungsgemäßen Verfahren im Vergleich zu herkömmlichen Transfektionsverfahren. Die Bezeichnung "MK-7-11" ist die Bezeichnung für das im folgenden Beispiel erhaltene Polymer aus Triethylentetramin und Ethylenglykoldiglycidylether.

### BEISPIEL (TRANSFORMATION EINER ZELLE)

### Herstellung des Polymers

Triethylentetramin (10 mmol) und Ethylenglykoldiglycidylether (10 mmol) werden in 50 ml Dimethylsulfoxid gelöst und die Lösung in einem Rundkolben für 3 Stunden auf 60°C temperiert. Anschließend wurde die Reaktionsmischung mit dem gleichen Volumenteil vollentsalztem Wasser auf das doppelte verdünnt und gegen vollentsalztes Wasser für 18 Stunden dialysiert, wobei dreimal das Wasser gewechselt wurde (Dialysemembran mit einem *"molecular weight cut off"* von 1 kDa). Nach der Dialyse wurde die Probe gefriergetrocknet, wobei eine wasserklare, viskose Substanz erhalten wurde. Diese wurde in endotoxinfreiem, entsalztem Wasser gelöst, wobei eine Polymer-Konzentration von 3 mg/ml erhalten wurde. Die Sterilisation der so erhaltenen, wässrigen Polymerlösung erfolgte mittels Sterilfiltration.

### Kultivierung der Zellen

Die Hepatomazellinien Huh-7 wurde in einer 96-Well-Platte in einer Dichte von 2 × 10⁴ Zellen/Well in je 100 µl DMEM-Medium pro Well ausplattiert. 24 Stunden nach Aussaat der Zellen wurde die Transfektion durchgeführt.

### Bildung des Komplexes

Zur Bildung des Komplexes aus Nukleinsäure und Polymer wurden die in der Tabelle 1 angegebenen Mengen an DNA (pCMVβ-Plasmid, Clontech Laboratories), gelöst in DMEMS-Medium (0.01µg/µl*) sowie die in der Tabelle 1 angegebenen Mengen der wässrigen, sterilen Lösung des aus Triethylentetramin und Ethylenglykoldiglycidylether erhaltenen Polymers ("MK-7-11") in die Vertiefung eines 96-Wells pipettiert und für 10 Minuten bei Raumtemperatur inkubiert. Als Kontrolle wurde eine Transfektion mit dem aus dem Stand der Technik bekannten Transfektionsreagenz SuperFect^{®} der Firma Qiagen GmbH, Hilden, Deutschland, durchgeführt.

*Anmerkungen zur Vorgehensweise: Die Komplexbildung zwischen der DNA und dem Polymer erfolgte in 50µl Kulturmedium ohne Serum. Die Konzentrationsangabe bezieht sich auf dieses Volumen. Im Anschluss an die Inkubationszeit von 10 Minuten wurden 100µl Kulturmedium mit Serum hinzu pipettiert. Das gesamte Volumen (150µl) wurde anschließend auf die Zellen gegeben, von denen zuvor das Kulturmedium entfernt wurde.

**Tabelle 1**

| Komplex | Menge an Plasmid | Menge an Polymer | Menge an Superfect^{®} |
|---|---|---|---|
| 1 | 0,5 µg | 0,5 µl | |
| 2 | 0,5 µg | 1 µl | |
| 3 | 0,5 µg | 2 µl | |
| 4 | 0,5 µg | 4 µl | |
| 5 | 0,5 µg | | 0,5µl |
| 6 | 0.5 µg | | 1 µl |
| 7 | 0,5 µg | | 2 µl |
| 8 | 0,5 µg | | 4 µl |

### Transfektion der Zellen

Zur Transfektion wurden die Komplexe zu den Zellen gegeben und für 4 Stunden bei 37°C inkubiert, wobei unmittelbar zuvor ein Mediumwechsel durchgeführt wurde. Nach der Inkubationszeit von 4 Stunden wurde ein Mediumwechsel durchgeführt, wobei die komplexhaltige Lösung durch frisches Zellkulturmedium ersetzt wurde. Nach Inkubation der Zellen mit den erfindungsgemäßen Komplexen bzw. mit den Komplexen aus Nukleinsäure und SuperFect wurden die Zellen an Tag 2 nach der Transfektion mittels eines Lysepuffers (5 mM MgCl₂, 1% NP-40, 100 mM NaCl, 10 mM Tris/HCl, pH 7,4) lysiert. Die Transfektionseffizienz wurde mittels eines β-Galaktosidase-Assays mit ONPG (2-Nitrophenyl-β-D-galaktopyranosid, Merck KGaA, Darmstadt, Deutschland) als Substrat bestimmt. Die Transfektionseffizienz (in Units β-Galaktosidase pro ml) ist in der Figur 1 dargestellt.

Aus der Figur 1 ist zu entnehmen, dass mittels des erfindungsgemäßen Verfahrens bessere Transfektionseffizienzen erzielt werden können als mit den aus dem Stand der Technik bekannten Transformationsreagenzien.

## Patentansprüche

1. Ein Verfahren zum Einschleusen eines Biomoleküls in eine Zelle in vitro, beinhaltend die Verfahrensschritte:
i) Herstellen eines Komplexes aus einem Biomolekül, vorzugsweise einer Nukleinsäure, und einem Polymer, welches erhältlich ist durch Reaktion eines Amin-Monomers, aufweisend mindestens zwei Amin-Gruppen, mit einem Epoxid-Monomer, aufweisend mindestens zwei Epoxid-Gruppen, sowie
ii) Einschleusen des Biomoleküls in eine Zelle durch das in Kontakt bringen einer Zelle mit dem Komplex.

2. Verfahren nach Anspruch 1, wobei die Herstellung des Komplexes im Verfahrensschritt i) durch das in Kontakt bringen des Polymers mit dem Biomolekül bei einem pH-Wert in einem Bereich von 6 bis 8 erfolgt.

3. Verfahren nach einem der vorhergehenden Ansprüche, wobei das Biomolekül eine Desoxyribonukleinsäure ist.

4. Verfahren nach einem der vorhergehenden Ansprüche, wobei das Amin-Monomer mindestens zwei funktionelle Gruppen der Struktur I 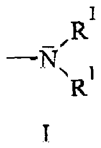 aufweist, worin die Reste R¹ innerhalb der funktionellen Gruppe gleich oder verschieden sein können und einen C₁- bis C₂₀-Kohlenwasserstoff-Rest, einen hydroxyfunktionellen C₁- bis C₂₀-Kohlenwasserstoff-Rest oder ein Wasserstoffatom darstellen.

5. Verfahren nach einem der vorhergehenden Ansprüche, wobei das Amin-Monomer die Struktur II 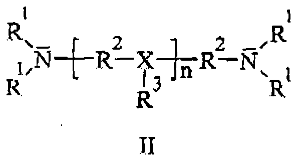 aufweist, in der
n eine ganze Zahl aus dem Bereich von 1 bis 100 ist,
X sofern vorhanden, ein Sauerstoffatom oder ein Stickstoffatom ist, wobei im Falle eines Sauerstoffatoms die R³-Gruppe am Atom X nicht vorhanden ist,
R¹ innerhalb der Struktur II gleich oder verschieden sein kann und einen C₁- bis C₂₀-Kohlenwasserstoff-Rest, einen hydroxyfunktionellen C₁- bis C₂₀-Kohlenwasserstoff-Rest oder ein Wasserstoffatom darstellt,
R² innerhalb der Struktur II gleich oder verschieden sein kann und eine C₁-bis C₂₀-Alkylen-Gruppe oder eine C₁-bis C₂₀₀-Oxyalkylen-Gruppe darstellt, und
R³ innerhalb der Struktur II gleich oder verschieden sein kann und einen C₁-Kohlenwasserstoff-Rest, einen hydroxyfunktionellen C₁- bis C₂₀-Kohlenwasserstoff-Rest, ein Wasserstoffatom oder einen Rest der Struktur III 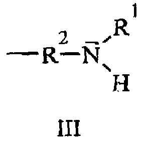
darstellt.

6. Verfahren nach einem der vorhergehenden Ansprüche, wobei das Amin-Monomer ein aromatisches Ringsystem umfasst, an das mindestens zwei funktionelle Gruppen der Struktur I 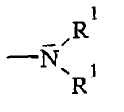 gebunden sind, worin R¹ einen C₁- bis C₂₀-Kohlenwasserstoff-Rest, einen hydroxyfunktionellen C₁- bis C₂₀-Kohlenwasserstoff-Rest oder ein Wasserstoffatom darstellt.

7. Verwendung eines Polymers, welches erhältlich ist durch Reaktion eines Amin-Monomers, aufweisend mindestens zwei Amino-Gruppen, und eines Epoxid-Monomers, aufweisend mindestens zwei Epoxid-Gruppen, zum Einschleusen eines Biomoleküls in eine Zelle in vitro.

8. Ein Kit zum Einschleusen eines Biomoleküls in eine Zelle, umfassend
(β1) das in den Ansprüchen 4 bis 6 definierte Polymer, welches erhältlich ist durch die Reaktion des Amin-Monomers, aufweisend mindestens zwei Amin-Gruppen, mit dem Epoxid-Monomer, aufweisend mindestens zwei Epoxid-Gruppen, sowie
(β2) weitere Kit-Komponenten ausgewählt aus der Gruppe umfassend: Protaminsulfat aus Lachssperma, PBS, physiologische Kochsalzlösung und/oder liposomale oder nicht liposomale Lipide.

9. Verwendung des Kits nach Anspruch 8 in einem Verfahren nach einem der Ansprüche 1 bis 6.

10. Ein Komplex aus einem Biomolekül, vorzugsweise einer Nukleinsäure, und einem Polymer, welches erhältlich ist durch Reaktion eines Amin-Monomers, aufweisend mindestens zwei Amin-Gruppen, mit einem Epoxid-Monomer, aufweisend mindestens zwei Epoxid-Gruppen.

11. Eine therapeutische Zusammensetzung, beinhaltend einen Komplex aus einem Biomolekül, vorzugsweise einer Nukleinsäure, und einem Polymer, welches erhältlich ist durch Reaktion eines Amin-Monomers, aufweisend mindestens zwei Amin-Gruppen, mit einem Epoxid-Monomer, aufweisend mindestens zwei Epoxid-Gruppen.

12. Verwendung eines Komplex aus einem Biomolekül, vorzugsweise einer Nukleinsäure, und einem Polymer, welches erhältlich ist durch Reaktion eines Amin-Monomers, aufweisend mindestens zwei Amin-Gruppen, mit einem Epoxid-Monomer, aufweisend mindestens zwei Epoxid-Gruppen, zur Herstellung einer therapeutischen. Zusammensetzung.

## Claims

1. An in vitro method for transferring a biomolecule into a cell, containing the steps:
i) production of a complex from a biomolecule, preferably a nucleic acid, and a polymer, which is obtainable by reaction of an amine monomer, having at least two amine groups, with an epoxide monomer, having at least two epoxide groups, and
ii) transferring the biomolecule into a cell by contacting a cell with the complex.

2. The method as claimed in claim 1, **characterized in that** the preparation of the complex in step i) is carried out by contacting the polymer with the biomolecule at a pH value in a range from 6 to 8.

3. The method as claimed in one of the preceding claims, **characterized in that** the biomolecule is a deoxyribonucleic acid.

4. The method as claimed in one of the preceding claims, **characterized in that** the amine monomer has at least two functional groups of structure I 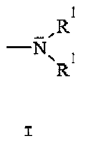 in which the residues R¹ within the functional group can be identical or different and represent a C₁ to C₂₀ hydrocarbon residue, a hydroxyfunctional C₁ to C₂₀ hydrocarbon residue or a hydrogen atom.

5. The method as claimed in one of the preceding claims, **characterized in that** the amine monomer has the structure II 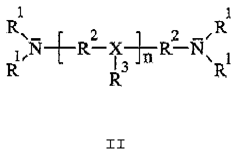 in which
n is an integer in the range from 1 to 100,
X if present, is an oxygen atom or a nitrogen atom, and in the case of an oxygen atom the R³ group is not present on atom X,
R¹ within structure II can be identical or different and represents a C₁ to C₂₀ hydrocarbon residue, a hydroxyfunctional C₁ to C₂₀ hydrocarbon residue or a hydrogen atom,
R² within structure II can be identical or different and represents a C₁ to C₂₀ alkylene group or a C₁ to C₂₀₀ oxyalkylene group, and
R⁵ within structure II can be identical or different and represents a C₁ to C₂₀ hydrocarbon residue, a hydroxyfunctional C₁ to C₂₀ hydrocarbon residue, a hydrogen atom or a residue of structure III

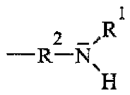

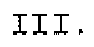

6. The method as claimed in one of the preceding claims, **characterized in that** the amine monomer comprises an aromatic ring system, to which at least two functional groups of structure I 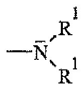 are bound, in which R¹ represents a C₁ to C₂₀ hydrocarbon residue, a hydroxyfunctional C₁ to C₂₀ hydrocarbon residue or a hydrogen atom.

7. In-Vitro-Use of a polymer, which is obtainable by reaction of an amine monomer, having at least two amino groups, and an epoxide monomer, having at least two epoxide groups, for transferring a biomolecule into a cell.

8. A kit for transferring a biomolecule in a of cells, comprising
(β1) the polymer defined in claims 4 to 17, which is obtainable by the reaction of the amine monomer, having at least two amine groups, with the epoxide monomer, having at least two epoxide groups, and
(β2) other kit components, selected from the group comprising protamine sulfate from salmon sperm, PBS, physiological saline solution and/ or liposomal or nonliposomal lipids.

9. Use of the kit as claimed in claim 8 in a method as claimed in one of claims 1 to 6.

10. A complex of a biomolecule, preferably a nucleic acid, and a polymer, which is obtainable by reaction of an amine monomer, having at least two amine groups, with an epoxide monomer, having at least two epoxide groups.

11. A therapeutic composition, containing a complex of a biomolecule, preferably a nucleic acid, and a polymer, which is obtainable by reaction of an amine monomer, having at least two amine groups, with an epoxide monomer, having at least two epoxide groups.

12. Use of a complex of a biomolecule, preferably a nucleic acid, and a polymer, which is obtainable by reaction of an amine monomer, having at least two amine groups, with an epoxide monomer, having at least two epoxide groups, for the production of a therapeutic composition.

## Revendications

1. Procédé pour transfecter in vitro une biomolécule dans une cellule, comprenant les étapes suivantes :
i) fabrication d'un complexe à partir d'une biomolécule, de préférence un acide nucléique, et d'un polymère qui peut être obtenu par réaction d'un monomètre amine, comportant au moins deux groupes amines, avec un monomère époxyde, comportant au moins deux groupes époxydes, ainsi que
ii) transfection de la biomolécule dans une cellule par mise en contact d'une cellule avec le complexe.

2. Procédé selon la revendication 1, la fabrication du complexe dans l'étape de procédé i) s'effectuant par mise en contact du polymère avec la biomolécule pour une valeur de pH comprise entre 6 et 8.

3. Procédé selon l'une des revendications précédentes, la biomolécule étant un acide désoxyribonucléique.

4. Procédé selon l'une des revendications précédentes, le monomère amine comportant au moins deux groupes fonctionnels de la structure I : 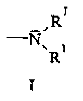 les radicaux R¹ à l'intérieur du groupe fonctionnel pouvant être identiques ou différents et représentant un radical hydrocarbure C₁ à C₂₀, un radical hydrocarbure C₁ à C₂₀ hydroxyfonctionnel ou un atome d'hydrogène.

5. Procédé selon l'une des revendications précédentes, le monomère amine comportant la structure II : 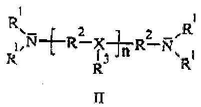 dans laquelle
n est un nombre entier compris entre 1 et 100,
X est, dans la mesure où il est présent, un atome d'oxygène ou un atome d'azote, le groupe R³ sur l'atome X étant absent dans le cas d'un atome d'oxygène,
R¹ à l'intérieur de la structure II pouvant être identique ou différent et représentant un radical hydrocarbure C₁ à C₂₀, un radical hydrocarbure C₁ à C₂₀ hydroxyfonctionnel ou un atome d'hydrogène,
R² à l'intérieur de la structure II pouvant être identique ou différent et représentant un groupe alkyle C₁ à C₂₀ ou un groupe oxyalkyle C₁ à C₂₀₀,
R³ à l'intérieur de la structure II pouvant être identique ou différent et représentant une radical hydrocarbure C₁ à C₂₀, un radical hydrocarbure C₁ à C₂₀ hydroxyfonctionnel, un atome d'hydrogène ou un radical de la structure III

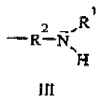

6. Procédé selon l'une des revendications précédentes, le monomère amine comprenant un système d'anneau aromatique auquel sont liés au moins deux groupes fonctionnels de la structure I 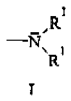 R¹ représentant un radical hydrocarbure C₁ à C₂₀, un radical hydrocarbure C₁ à C₂₀ hydroxyfonctionnel ou un atome d'hydrogène.

7. Utilisation d'un polymère, lequel peut être obtenu par réaction d'un monomère amine, comportant au moins deux groupes amines, et d'un monomère époxyde, comportant au moins deux groupes époxydes, pour la transfection in vitro d'une biomolécule dans une cellule.

8. Kit pour la transfection d'une biomolécule dans une cellule, comprenant :
(β1) le polymère défini dans les revendications 4 à 6, qui peut être obtenu par la réaction du monomère amine, comportant au moins deux groupes amines, avec le monomère époxyde, comportant au moins deux groupes époxydes, ainsi que
(β2) d'autres composants de kit choisis dans le groupe comprenant : du sulfate de protamine extrait de sperme de saumon, du PBS, du sérum physiologique et/ou des lipides de formule liposomale ou non.

9. Utilisation du kit selon la revendication 8 dans un procédé selon l'une des revendications 1 à 6.

10. Complexe constitué d'une biomolécule, de préférence un acide nucléique, et d'un polymère, qui peut être obtenu par réaction d'un monomère amine, comportant au moins deux groupes amines, avec un monomère époxyde, comportant au moins deux groupes époxydes.

11. Composé thérapeutique, contenant un complexe constitué d'une biomolécule, de préférence un acide nucléique, et d'un polymère, qui peut être obtenu par réaction d'un monomère amine, comportant au moins deux groupes amines, avec un monomère époxyde, comportant au moins deux groupes époxydes.

12. Utilisation d'un complexe constitué d'une biomolécule, de préférence un acide nucléique, et d'un polymère, qui peut être obtenu par réaction d'un monomère amine, comportant au moins deux groupes amines, avec un monomère époxyde, comportant au moins deux groupes époxydes, pour la fabrication d'un composé thérapeutique.
